# FASCICULE DE BREVET EUROPEEN

(11) **EP 0 960 132 B1**
(45) Date de publication et mention de la délivrance du brevet: **05.06.2002**
(21) Numéro de dépôt: 98908175.7
(22) Date de dépôt: 12.02.1998
(51) Int. Cl.: C08B 37/00, C12P 19/04

(54) **POLYSACCHARIDE, MICRO-ORGANISME ET PROCEDE POUR SON OBTENTION, COMPOSITION LE CONTENANT ET APPLICATION**
POLYSACCHARID, MIKROORGANISMUS UND VERFAHREN ZU DESSEN HERSTELLUNG, DIESES ENTHALTENDE ZUSAMMENSETZUNG UND VERWENDUNG
POLYSACCHARIDE, MICRO-ORGANISM AND METHOD FOR OBTAINING SAME, COMPOSITION CONTAINING IT AND APPLICATION

(30) Priorité: 12.02.1997 FR 9701624
(43) Date de publication de la demande: 01.12.1999
(73) Titulaire: Agro Industrie Recherches et Développement ( A.R.D.) Société Anonyme, 51110 Pomacle (FR); CENTRE NATIONAL DE LA RECHERCHE SCIENTIFIQUE (CNRS), 75794 Paris Cedex 16 (FR)
(72) Inventeur: ALAMI, Younes,, 13090 Aix en Provence (FR); HEULIN, Thierry, 13100 Aix en Provence (FR); MILAS, Michel, 38320 Herbeys (FR); DE BAYNAST, Régis, 78000 Versailles (FR); HEYRAUD, Alain, 38113 Veurey-Voroize (FR); VILLAIN, Agnès, 38000 Grenoble (FR)
(74) Mandataire: Hammond, William
(86) Numéro de dépôt international: FR9800269
(87) Numéro de publication internationale: WO9835993

(56) Documents cités:
- EP-A- 0 534 855
- GB-A- 2 223 503
- DATABASE WPI Week 9019 Derwent Publications Ltd., London, GB; AN 90-145127 XP002045240 & JP 02 092 901 A (NAKANO VINEGAR CO LTD) , 3 avril 1990
- PATENT ABSTRACTS OF JAPAN vol. 007, no. 235 (C-191), 19 octobre 1983 & JP 58 127701 A (BAIORISAACHI CENTER:KK), 29 juillet 1983,

## Description

La présente invention concerne un nouveau polysaccharide, micro-organisme et procédé pour son obtention, composition le contenant et application.

On sait que les populations microbiennes constituent un large réservoir pour la mise en oeuvre de nouvelles molécules. La description des bactéries présentes dans les rhizosphères de plantes céréalières a d'abord permis ds mettre en évidence des espèces fixatrices d'azote pour la plupart associées aux racines de blé, tournesol, riz et maïs. L'isolement de ces espèces a nécessité la mise au point de méthodes d'isolement spécifiques : « le modèle spermosphère » permettant de sélectionner les bactéries les plus adaptées à la rhizosphère et l'immunopiégeage utilisant les anticorps spécifiques de certaines espèces. Ces bactéries sont aussi impliquées dans les mécanismes d'attachement et de colonisation des racines.

Depuis plusieurs années, les recherches se sont focalisées sur la capacité de la plupart des bactéries, présentes à la surface des racines et dans la rhizosphère, à produire des exopolysaccharides (EPS). Il a largement été démontré que ces polymères jouent un rôle dans la colonisation des racines par les bactéries et l'agrégation des sols autour des racines.

De récents travaux s'intéressent à l'étude de souches pour leur capacité à stimuler l'agrégation du sol autour des racines de tournesol et de blé.

Un objet de la présente invention est de fournir un micro-organisme produisant des exopolysaccharides sur milieu gélosé, à croissance rapide sur des milieux de culture peu exigeants à base de matières premières agricoles (hydrolysats de son de blé, peptides de blé, sirops de glucose, hydrolysats de co-produits d'amidonnerie par exemple), faciles à mettre en oeuvre et bien entendu non-pathogènes et génétiquement stables.

Ainsi, a été mise en évidence, une souche dont la cartographie et le séquençage du gène codant pour l'ARNr 16S indiquent qu'elle appartient à la famille des Rhizobiacées (subdivision alpha des Protéobactéries). Le pourcentage de similarité des acides nucléiques de cette région du chromosome (ADNr 16S) de la souche YAS34 est de 97,2 % avec *Rhizobium etli* et de 96,2 % avec *Rhizobium leguminosarum* (souche LMG 9518). Une hybridation sur colonies de YAS34 avec une sonde oligonucléotidique spécifique des *Rhizobium (sensu lato*) dans le gène nodD s'est révélée positive (communication personnelle G. Laguerre, INRA, Dijon). La présence du gène nodD et les pourcentages de similarité du gène codant pour l'ARNr 16S avec des espèces appartenant au genre *Rhizobium* sont autant d'éléments permettant d'affirmer que la souche YAS34 est un *Rhizobium*.

L'empreinte génotypique de la souche YAS34 par rep-PCR en utilisant trois différents jeux d'amorce (REP, ERIC et BOX) est disponible.

YAS34 a été déposée sous le n° I-1809 à la CNCM de l'Institut Pasteur le 15 janvier 1997.

Cette souche YAS 34 a été isolée de la surface des racines (rhizoplan) d'un tournesol (Helianthus annuus cv Albena) prélevé au stade quatre feuilles. Les semences utilisées n'avaient pas été traitées à l'aide de produits phytosanitaires et avaient été stérilisées avant le semis. Le sol était de type limoneux dont le cation échangeable majoritaire est le calcium.

Cette souche YAS 34 est une bactérie Gram négative, aérobie catalase positive et oxydase négative. Il s'agit d'un bâtonnet mobile formant des colonies élastiques, translucides de couleur blanche sur milieu RCV-glucose (4 g/l).

La souche YAS 34 réalise la biosynthèse de polysaccharide par fermentation en présence d'un milieu de culture contenant une source de carbone préférentiellement assimilée.

Il a été mis en évidence que les sources carbonées, les plus performantes en terme de croissance et de production de polysaccharides, sont le glucose, le fructose, le saccharose et le galactose.

Cette aptitude à assimiler de nombreux glucides a conduit à étudier la capacité fermentaire de cette souche sur différents milieux d'origine végétale issus du fractionnement de matières agricoles comme le blé, la pomme de terre et le raisin. Dans le tableau I ci-après, sont regroupés les différents milieux testés, tandis que le tableau Il indique les résultats obtenus.

**TABLEAU I**

| **PRODUITS AGRICOLES** | **MILIEUX TESTES** | **COMPOSITIONS SUCRES ET MATIERES AZOTEES** |
|---|---|---|
| Pomme de terre | JCII ED : jus clair II électrodialysé | |
| Son de Blé | JPAS : jus de presse après saccharification | |
| | FAM : filtrat après microfiltration | Glucose : 12 à 15 g/L Matière azotée : 5,25 g/L |
| Raisin | JRB 033 ED LD : jus de presse de marc de raisin après électrodialyse | Glucose : 10 g/L Fructose : 10 g/L Matière azotée : traces |

**TABLEAU II**

| **SUBSTRATS NATURELS** | **µ max (h-1)** | **DO à 600 nm max** | **VISCOSITE (mPa.s à 25°C, 26 s-1)** | | **CONSOMM. TOTALE GLUCOSE (g/L)** |
|---|---|---|---|---|---|
| | | | **à 52 h** | **à 140 h** | |
| **Pomme de terre** | | | | | |
| JCII ED | 0,21 | 7,6 | 238 | 273 | 5,6 |
| JCII ED1/2 | 0,26 | 6,04 | 197 | 193 | 3,8 |

| **Son de Blé** | | | | | |
|---|---|---|---|---|---|
| JPAS 2/3 | 0,17 | 3,9 | 267 | 374 | 9,5 |
| FAM | 0,47 | 2,9 | 280 | 356 | 5,6 |

| **Marc de raisin** | | | | | |
|---|---|---|---|---|---|
| JRB 03 ED LD1/2 | nd | | 9 | 36 | 1,8 |

Le milieu dénommé FAM issu du son de blé, qui renferme simultanément 12 g/L de glucose et 5,25 g/L de matière azotée, s'avère être le plus performant à la fois pour la croissance de la souche (µ max 0,47 h-1) et la biosynthèse de polysaccharide (viscosité proche de 400 mPa.s en fin de culture).

Il a été étudié différents milieux de synthèse dont notamment ceux de composition ci-après :

| RCVs | |
|---|---|
| Glucose | 20 g |
| Extrait de levure | 1,72 g |
| Solution tampon (2) | 15 ml |
| Solution minérale (1) | 50 ml |
| Eau osmosée | qsp 1 l |

| DSM | |
|---|---|
| Glucose | 20 g |
| Corn steep | 5 g |
| NaNO3 | 2 g |
| K₂HPO₄ | 1 g |
| MgSO₄, 7H₂O | 1,5 g |
| Solution E (3) | 2,5 ml |
| Eau osmosée | qsp 1 l |

Composition des solutions minérale (1), tampon (2) et E (3)

| (1) Solution minérale | |
|---|---|
| EDTA (tritriplex II) | 0,4 g |
| MgSO₄, 7H₂O | 2 g |
| CaCl₂, 2H₂O | 2 g |
| FeSO₄, 7H₂O | 0,44 g |
| Solution éléments | 20 ml |
| Eau osmosée | qsp 1 l |

| Solutions éléments | |
|---|---|
| ZnSO₄,7H₂O | 430 mg |
| MnSO₄, 7H₂O | 1300 mg |
| Na₂MoO₄, 2H₂O | 750 mg |
| H₃BO₃ | 2800 mg |
| CuSO₄, 5H₂O | 22,5 mg |
| CoSO₄, 7H₂O | 70 mg |
| Eau osmosée | qsp 1 l |

| (2) Solution tampon | |
|---|---|
| KH₂PO₄ | 40 g |
| K₂HPO₄ | 60 g |
| Eau osmosée | qsp 1 l |

| (3) Solution E | |
|---|---|
| CaCl₂, 2H₂O | 3 g |
| FeIII nitrate | 1 g |
| MnSO₄ | 0,2 g |
| ZnCl₂ | 0,1 g |
| CuSO₄. 5H₂O | 0,025 g |
| Na₂B₄O₇, 10H₂O | 0,02 g |
| CaCl₂ | 0,004 g |
| Na₂MoO₄, 2H₂O | 0,01 g |
| Eau osmosée | qsp 1 l |

| NA | |
|---|---|
| Extrait de viande | 3 g |
| Peptone | 5 g |
| Eau osmosée | qsp 1 l |

Le tableau III regroupe les résultats obtenus.

**TABLEAU III**

| **Milieu** | **Rapport C/N** | **DO à 600 nm max** | **µ max (h-1)** | **Viscosité finale à 26s-1, 20°C (mPa.s)** |
|---|---|---|---|---|
| RCV s | 50 | 2,70 | 0,28 | 227 |
| DSM+ | 8 | 6,20 | 0,38 | 254 |
| NA | 0,1 | 1,91 | 0,35 | 7 |

On peut remarquer d'après ce tableau III que les milieux riches en azote et carencés en carbone (C/N faible) tel que le milieu NA favorisent la croissance de la souche mais ne permettent pas la production de polysaccharides. Par contre, les milieux RCV et DSM riches en substrats azotés et carbonés, réalisent le meilleur compromis en permettant à la fois une bonne croissance de la souche et la synthèse de polysaccharides. Ces essais ont également mis en évidence qu'il est possible de dissocier la croissance de la souche de la production de polysaccharides.

Il a été ainsi déterminé un milieu de préculture optimisé pour la croissance de la souche, dont la composition est pour un inoculum de 7,5 %.

| Opt2_ns | |
|---|---|
| Glucose (stérilisé séparément) | 20 g |
| Extrait de levure | 2,5 g |
| Sulfate d'ammonium | 1 g |
| Sol. Minérale (1) | 70 ml |
| Sol. Tampon (2) | 20 ml |
| Eau osmosée | qsp 1 l |
| Inocuium | 7,5 % |

De même, il a été étudié la mise au point d'un milieu optimisé pour la production d'exopolysaccharide, afin de maximiser la productivité en polymère. La composition, ci-après dénommée MP1, a donc été définie comme la plus performante.

| MP1 | |
|---|---|
| Glucose | 20 g |
| Extrait de levure | 1,7 g |
| Sol. Minérale (1) | 70 ml |
| Eau osmosée | qsp 1 l |

Dans le tableau IV, on a regroupé les résultats de croissance et de production en utilisant le milieu RCVs seul (référence) ou les deux milieux optimisés ci-dessus Opt2_ns et MP1.

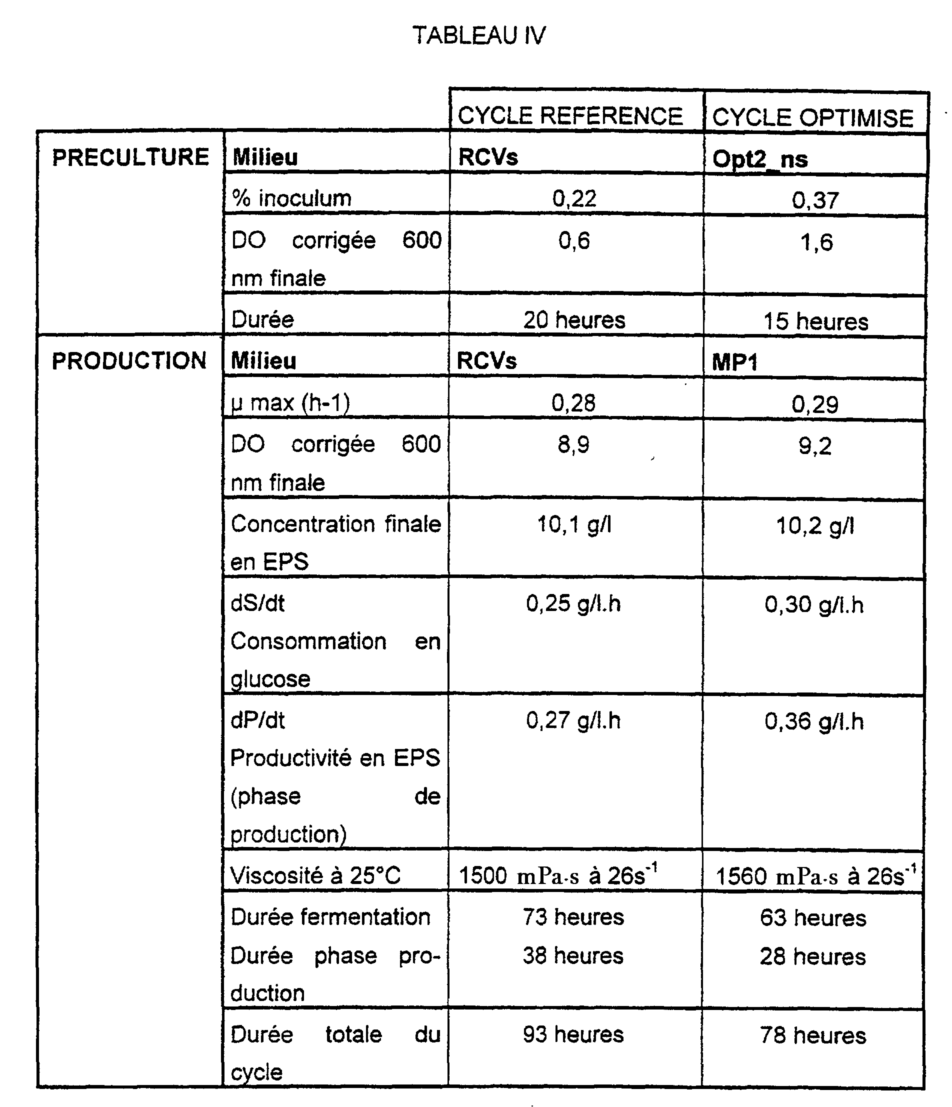

Pour récupérer le polysaccharide produit par la souche par fermentation comme explicité ci-dessus, deux méthodes peuvent être utilisées.

Selon une première méthode, le moût brut est soumis à une précipitation à l'éthanol puis à un séchage sous vide pour obtenir un produit sec contenant le polysaccharide brut. Le produit obtenu, remis en solution à 1 %, a des propriétés viscosifiantes. Cette solution, chauffée à une température comprise entre 70 et 95°C (de préférence entre 85 et 95°C), présente les mêmes propriétés gélifiantes que la solution préparée à partir du polysaccharide purifié, qui est obtenu selon la seconde méthode ci-après.

Selon cette seconde méthode, on soutire le moût de fermentation et on le dilue entre 1 et 1/20, de préférence entre 1 et 1/10. Puis, on porte la solution obtenue à une température comprise entre 70 et 95°C ; de préférence, ce traitement thermique est réalisé, entre 85 et 95°C. En effet, un traitement thermique à une température de 90°C provoque une liquéfaction du moût de fermentation, même après refroidissement à température ambiante. Pour un traitement à 90°C pendant environ une heure, la température de gélification ou refroidissement est inférieure à 20°C.

Si le traitement thermique est réalisé à une température inférieure à 90°C, celui-ci conduit après refroidissement à l'obtention d'un produit plus visqueux que le moût initial.

Ainsi, il apparaît que de tels traitements thermiques permettent de faciliter la séparation cellules/polymères, notamment par centrifugation.

On centrifuge le produit issu du traitement thermique par exemple à 13000 g ou on le soumet à une filtration tangentielle.

Le surnageant ainsi obtenu est soumis à une filtration frontale à 0,2 µm sur un filtre à plaques. Une telle filtration frontale permet en effet d'obtenir un filtrat extrêmement pur présentant des densités optiques (DO) à 600 nm pratiquement nulles (Tableau V).

**TABLEAU V**

| **Seuil de coupure (µm)** | **DO à 600 nm du Filtrat** |
|---|---|
| 0,8 | 0,02 |
| 0,45 | 0,005 |
| 0,8 puis 0,45 | 0,007 |
| 0,8 puis 0,22 | 0,003 |
| 0,45 puis 0,22 | 0,002 |
| Pression appliquée : 2.10⁵ Pascals | |

Le filtrat récupéré est traité de façon connue : il est concentré, soumis à une précipitation à l'éthanol puis à un séchage sous vide pour obtenir un produit sec comprenant un exopolysaccharide purifié.

Ainsi qu'il a été dit plus haut, cet exopolysaccharide est nouveau tant par sa nature que par ses propriétés.

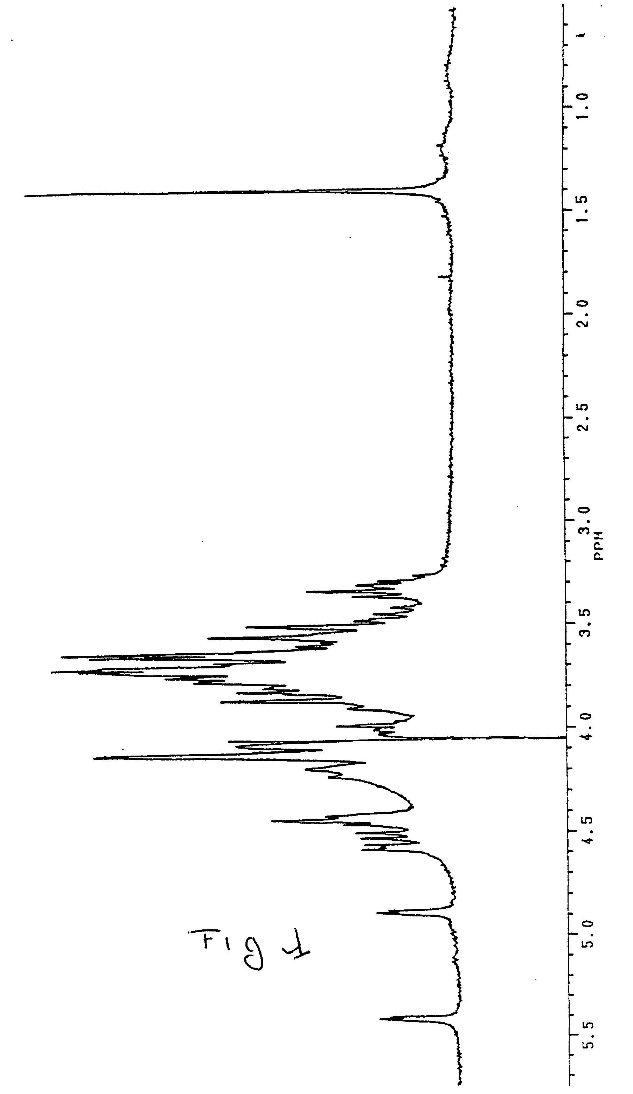

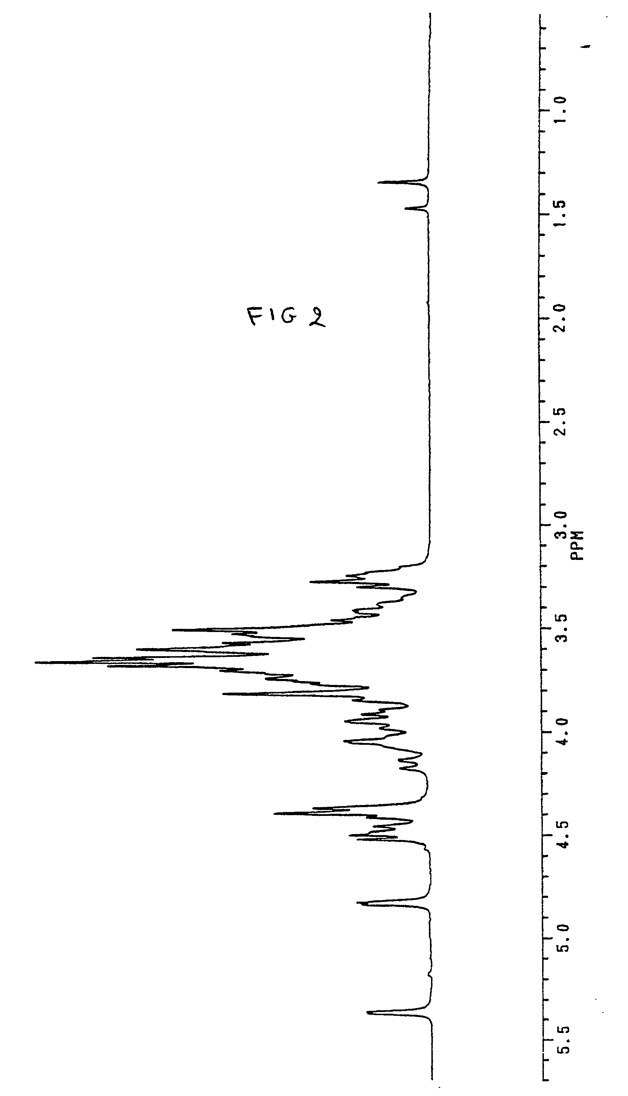

La détermination de la structure du polysaccharide ainsi obtenu par spectre RMN est l'objet des figures 1 et 2. Ceci a permis de déterminer que l'unité de répétition, qui possède une chaîne latérale, est composée majoritairement de 7 sucres
- 6 sucres neutres dont le glucose et le galactose
- 1 sucre acide

Il a été également mis en évidence la présence de substituants pyruvates et acétates. Les charges pyruvates et sucres acides confèrent à ce polysaccharide les propriétés de polyélectrolyte.

Lors de l'utilisation, pour des concentrations en polysaccharide de l'invention supérieures à 2 g/l, les solutions de ce dernier se transforment en gel. L'obtention du gel se fera par mise en solution dans l'eau ou dans toute solution aqueuse saline. Cette mise en solution est favorisée par un chauffage de la solution du polysaccharide de l'invention, de préférence supérieur à 60°C.

Le polysaccharide, selon la présente invention, présente en solution à 1 % une limpidité parfaite, de « qualité cristal », ce qui lui confère une position privilégiée par rapport à des produits connus tels que ceux commercialisés sous la marque AMIGEL® par la société A. MULLER, ou sous la dénomination commerciale CURDLAN par la société TAKEDA, les xanthanes commercialisées par la société KELCO et alginates de sodium proposés par la société SANOFI (cf. Tableau VI ci-après).

**TABLEAU VI**

| **Produit** | **Coloration DO à 420 nm** | **Trouble DO à 600 nm** | **Do à 860 nm** |
|---|---|---|---|
| Gel de l'invention | 0,060 | 0,031 | 0,020 |
| Amigel® | 1,200 | 0,958 | 0,742 |
| Curdlan | 1,232 | 1,601 | 1,629 |
| Xanthane LT | 0,302 | 0,187 | 0,124 |
| Alginates de sodium | 0,216 | 0,133 | 0,091 |

Par ailleurs, le polysaccharide selon l'invention présente par rapport à ceux connus une remise en solution la plus rapide (Tableau VII).

**TABLEAU VII**

| **Produit** | **Type de poudre** | **Durée de remise en solution** |
|---|---|---|
| Gel de l'invention | non mouillable | Qlq minutes |
| Amigel | mouillable | Qlq heures |
| Xanthane | non mouillable | Qlq dizaines de minutes |

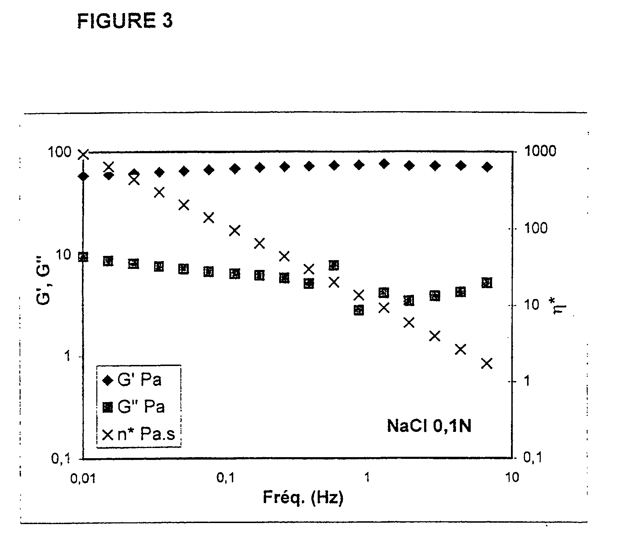

L'étude des propriétés mécaniques des gels de polysaccharide selon l'invention a permis de montrer la forte élasticité de ce gel : la figure 3 est relative à l'influence de la fréquence (Hz) sur les modules élastiques G' et de perte G" et sur la viscosité complexe n* d'un gel de polysaccharide selon l'invention (la concentration en polysaccharide est de 0,10 g/l et celle de NaCl de 0,1 M).

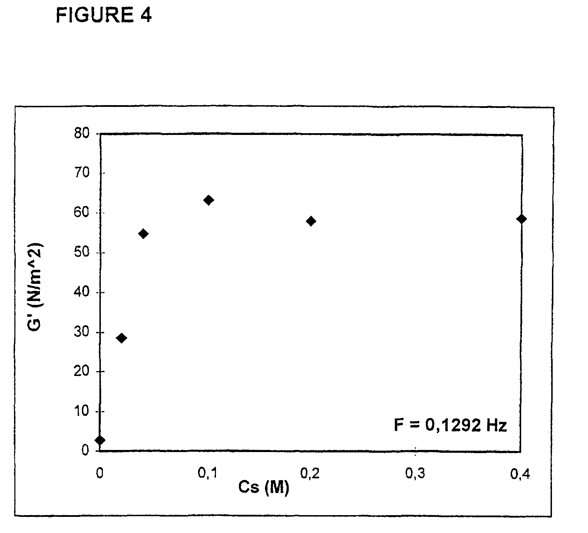

De même, il a été étudié l'incidence de la force ionique sur le module élastique des gels à 1% (poids/poids) en polysaccharide : la figure 4 est relative à l'influence de la concentration en sel (NaCl) sur le module élastique, mesuré à la fréquence de 0,13 Hz, d'un gel de polysaccharide selon l'invention à une concentration de 10 g/l.

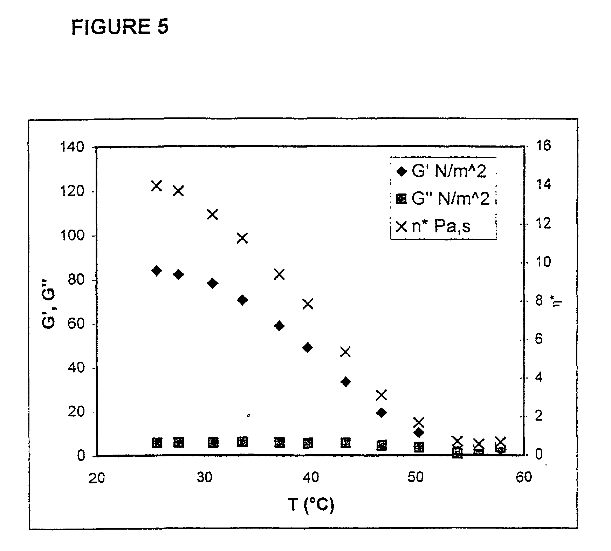

On constate qu'à partir d'une concentration en NaCl supérieure à 0,04 M, on obtient un gel élastique dont les caractéristiques ne varient quasiment plus avec la concentration en sel au moins jusqu'à 0,4 M. Des modules très voisins sont obtenus en présence de CaCl₂. Les gels formés sont thermoréversibles pour des traitements thermiques inférieurs à 90°C : la figure 5 est relative à l'influence de la température sur les modules élastiques G' et de perte G" et sur la viscosité complexe n* d'un gel de polysaccharide selon l'invention (la concentration en polysaccharide étant de 10g/l et celle en NaCl de 0,10 M).

Les résultats obtenus sont cependant fonction de la température et du temps de traitement, avec une possible destruction du gel pour des températures et/ou des temps de traitement trop élevés. La température de fusion du gel dépend très peu de la force ionique et de la nature des ions (Na⁺, Ca²⁺ par exemple).

Ce comportement rhéologique en milieu salé est extrêmement intéressant, car il offre des perspectives d'applications dans de nombreux secteurs de l'industrie reliés aux 3 milieux naturellement salés:
- le goût (env. 1,5 g sel/L) : domaine alimentaire,
- l'eau physiologique (env. 7,5 g sel/L) : secteur de l'agriculture, la cosmétique, la pharmacie,
- le milieu marin (env. 25 g sel/L) : industries pétrochimiques, cosmétique (gamme marine).

Afin d'illustrer ces différents domaines, des exemples d'application et de formulation du polysaccharide selon la présente invention sont indiqués ci-après sans toutefois présenter un caractère limitatif.

### I. Cosmétique

Le polysaccharide selon la présente invention trouve des applications en tant que:
- agent hydratant, seul ou en mélange avec des hydratants déjà connus, tels que l'acide hyaluronique, dans des crèmes et des laits,
- agent épaississant et agent de texture dans des lotions, des toniques, des crèmes et des laits (cosmétique blanche),
- agent de suspension et agent texturant dans des gels gommants, des filtres solaires,
- agent gélifiant dans des gels coiffants, des gels avant et après rasage, des gels lavants (shampooings et bains moussants).

Ci-après sont indiqués quelques types d'application.

### Crème hydratante (% pds/pds matière sèche)

- émulsifiant 4 %
- conservateur 0,5 %
- glycérol 5%
- polysaccharide de l'invention 0,25 %
- NaCl 0,5 %
- eau QSP 100 %

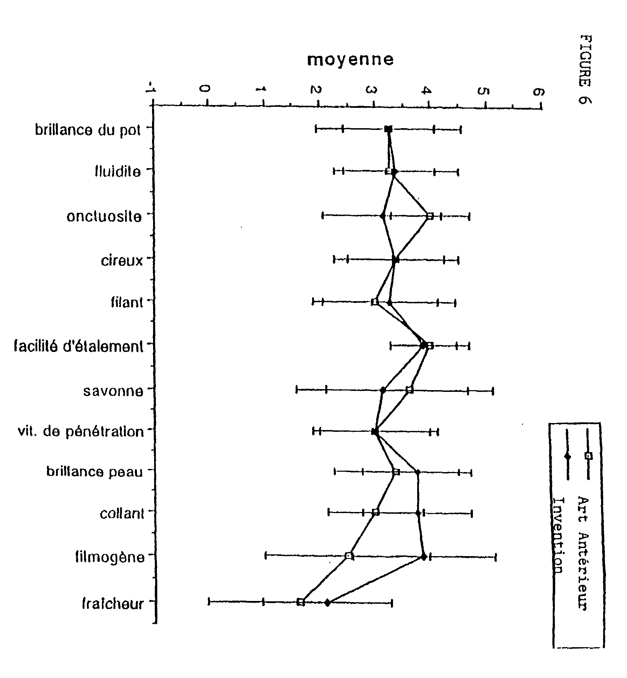

Pour cette application, le polysaccharide de l'invention apporte des propriétés particulièrement intéressantes par rapport à certains produits concurrents, au niveau de l'onctuosité, de la facilité d'étalement et de la fraîcheur. Des effets notables sur la diminution du pouvoir filtrant, du pouvoir collant, de l'effet filmogène, de la brillance de la peau et de la fluidité ont été observés, ainsi que le montre la figure 6.

### Lotion démaquillante (% pds/pds matière sèche)

- Dodécyl-tétradécyl galacturonate de sodium 0,5 %
- Hyaluronate de sodium 0,2 %
- Polysaccharide de l'Invention 0,4 %
- Eau de bleuet 5,0 %
- Conservateur QS
- Parfum, colorant QS
- Eau QSP 100,0 %

### Gel gommant (% pds/pds matière sèche)

- Polysaccharide de l'invention 0,75 %
- NaCl 0,5 %
- Carboxyméthylcellulose 0,5 %
- Noyau d'abricot broyé 2,0 %
- Conservateur, colorant, parfum QS
- Eau QSP 100,0 %

### Gel Lavant (% pds/pds matière sèche)

- Décyl-dodécyl galacturonate de sodium 4,0 %
- Lauryl bétaine 3,0 %
- Laureth(2)sulfate 3,0 %
- Acylat peptides (C12) 2,0 %
- Mono/oligoglycéride d'acide capric/caprilique éthoxylé 1,0 %
- Disodium lauryl sulfosuccinate 1,0 %
- Polysaccharide de l'invention 0,5 %
- NaCl 0,25 %
- Conservateur, parfum, colorant QS
- Eau QSP 100,0 %

### II-Détergents

Le polysaccharide selon la présente invention, trouve des applications en tant que :
* agent texturant et agent de suspension dans des crèmes et des gels récurants.
* agent gélifiant dans des gels déodorants et désinfectants.
* agent de texture dans des liquides vaisselle.

Dans le cas d'une crème récurante, une formulation possible est:
- Polysaccharide de l'invention 1,0 %
- NaCl 0,5 %
- Silicate d'aluminium calciné 25,0 %
- Sodium laurylsulfate 5,0 %
- Capryloamphopropionate 1,0 %
- Conservateur, colorant, parfum QS
- Eau QSP 100,0 %

### III-Alimentation

Le polysaccharide, selon la présente invention, trouve des applications en tant que :
* agent de texture, agent gélifiant et agent de suspension dans les desserts lactés, les vinaigrettes, les sauces (mayonnaise), les gelées et confitures, les aspics et terrines.
* agent texturant dans les boissons diététiques, les confiseries.

Par exemple, une formulation pour réaliser un flan chocolaté à froid ( % pds/pds matière sèche) est:
- Lait UHT 83,0 %
- Polysaccharide de l'invention 1,2 %
- NaCl 0,1 %
- Sucre 8,0 %
- Cacao 3,0 %
- Lait écrémé en poudre 2,0 %
- Huile végétale 0,7 %
- Arôme vanille QS
- Oeufs 2,0 %

### IV-Fermentation

Dans ce domaine, le polysaccharide, selon la présente invention, apporte la texture gélifiée à des milieux de culture gélosés semi-solides. Un exemple de formulation pour milieu gélosé semi-solide est en % pds/pds de matière sèche :
- Peptones 1 %
- Glucose 0,5 %
- NaCl 0,5 %
- Polysaccharide de l'invention 0,75 %
- Eau QSP 100 %

### V-Agriculture

En agriculture, le polysaccharide, selon la présente invention, trouve des applications en tant que :
* agent d'agrégation des sols,
* agent rétenteur d'eau, contribution au maintien du potentiel hydrique des sols et agent de protection de la sécheresse,
* agent d'enrobage de semences.

Ainsi, l'inoculation de la souche Rhizobium sur des semences de tournesol permet d'obtenir une forte colonisation des racines que le sol ait été ou non préalablement stérilisé (respectivement 90 % et 10 % de la microflore totale). Les conséquences de cette inoculation concernent l'augmentation de la masse de sol adhérant aux racines (+ 50 %) et la modification de la porosité de ce sol rhizosphérique (augmentation de la macroporosité). Ces résultats semblent indiquer que l'exopolysaccharide produit par la souche YAS 34, d'une part, contribue à l'assemblage des agrégats du sol (effet « collant ») et de ce fait augmente la fréquence des pores de transfert de l'eau vers la plante et, d'autre part, retient l'eau dans une phase polysaccharide gélifiée.

Pour confirmer le rôle rétenteur d'eau du polymère, des mélanges « sol + 1 % polysaccharide purifié » ont été réalisés. Les résultats montrent que à pF 2,5 (capacité au champ), le polysaccharide augmente de 50 % la rétention d'eau du sol et de la même façon, l'apport de polymère (1 %) se traduit par la nécessité d'appliquer deux fois plus d'énergie pour déshydrater le sol.

Les figures ci-après sont iointes en annexe :
- Figure 1 -: Polysaccharide de l'invention désacétylé. Spectre RMN¹H (300 MHz), solution dans D₂O, T = 358K
- Figure 2: - Polysaccharide de l'invention. Spectre RMN¹H (300 MHz), solution dans D₂O, T = 358K
- Figure 3 -: Influence de la fréquence sur les modules élastique (G') et de perte (G") et sur la viscosité complexe n* du gel du polysaccharide de l'invention. Concentration en polysaccharide 10 g/L, concentration en NaCl 0,1 M.
- Figure 4 -: Influence de la concentration en sel (NaCl) sur le module élastique, mesuré à la fréquence 0,13 Hz, d'un gel du polysaccharide de l'invention à la concentration de 10 g/L.
- Figure 5 -: Influence de la température sur les modules élastique (G') et de perte (G") et sur la viscosité complexe n* du gel du polysaccharide de l'invention. Concentration en polysaccharide 10 g/L, concentration en NaCl 0,1 M.
- Figure 6 -: Test sensoriel comparatif entre une formule de crème hydratante de l'art antérieur (art antérieur) et une formule de crème hydratante de l'invention (invention) comprenant 0,25 % d'un polysaccharide de l'invention et du chlorure de sodium.

Enfin, le polysaccharide, selon la présente invention, trouve aussi des applications en tant qu'agent texturant, agent épaississant et agent de suspension dans les peintures.

## Revendications

1. Polysaccharide ayant une unité de répétition qui possède une chaîne latérale et est constitué par six sucres neutres dont le glucose et le galactose et un sucre acide, des substituants pyruvates et acétates étant présents, une solution dans l'eau ou une solution saline à une concentration en ledit polysaccharide supérieure à 2 g/l formant un gel élastique et transparent.

2. Polysaccharide selon la revendication 1, **caractérisé par le fait que** la mise en solution dudit polysaccharide est favorisée par un chauffage de la solution à une température supérieure à 60°C.

3. Micro-organisme capable de synthétiser un polysaccharide selon l'une quelconque des revendications 1 à 2, **caractérisé par le fait qu'**il est un Rhizobium déposé sous le n° I-1809 auprès de la CNCM.

4. Procédé pour la production d'un polysaccharide selon la revendication 1, **caractérisé par le fait qu'**il comprend les étapes suivantes:
a) culture d'un micro-organisme qui est un Rhizobium déposé sous le n° I-1809 auprès de la CNCM sur un milieu riche en glucose et en matière azotée,
b) récupération du moût de fermentation,
c) obtention directe du polysaccharide brut par précipitation.

5. Procédé selon la revendication 4, **caractérisé par le fait que** le moût de fermentation issu de l'étape b) est traité comme suit:
d) traitement thermique dudit moût après dilution, à une température comprise entre 70 et 95°C,
e) centrifugation,
f) filtration du surnageant issu de l'étape d), par exemple, par filtration frontale
g) traitement du filtrat pour obtenir le polysaccharide sous forme sèche.

6. Procédé selon la revendication 4, **caractérisé par le fait qu'**à l'étape a), on utilise deux milieux de culture l'un dit de précuiture et l'autre dit de production.

7. Procédé selon la revendication 6, **caractérisé par le fait que** le milieu dit de préculture a pour composition:
| | |
|---|---|
| Glucose (stérilisé séparément) | 20 g |
| Extrait de levure | 2,5 g |
| Sulfate d'ammonium | 1 g |
| Solution Minérale (1) | 70 ml |
| Solution Tampon (2) | 20 ml |
| Eau osmosée | qsp 1 l |
| Inoculum | 7,5 % |

8. Procédé selon la revendication 6, **caractérisé par le fait que** le milieu dit de production a pour composition :
| | |
|---|---|
| Glucose | 20 g |
| Extrait de levure | 1,7 g |
| Sol. Minérale (1) | 70 ml |
| Eau osmosée | qsp 1 l |

9. Application du polysaccharide selon l'une des revendications 1 à 2, en tant qu'agent hydratant (seul ou en mélange avec d'autres hydratants déjà connus tels que l'acide hyaluronique), agent épaississant, agent gélifiant, agent de suspension dans le domaine de la cosmétique.

10. Application du polysaccharide selon l'une des revendications 1 à 2, en tant qu'agent gélifiant, agent de suspension dans le domaine de la détergence.

11. Application du polysaccharide selon l'une des revendications 1 à 2 en tant qu'agent texturant et agent de suspension dans les produits alimentaires.

12. Application du polysaccharide selon l'une des revendications 1 à 2 en tant qu'agent gélifiant dans des milieux de fermentation gélosés.

13. Application du polysaccharide selon l'une des revendications 1 à 2 en tant qu'agent d'agrégation des sols, agent rétenteur d'eau et agent d'enrobages de semences dans le domaine de l'agriculture.

14. Application du polysaccharide selon l'une des revendications 1 à 2 en tant qu'agent texturant, agent épaississant et agent de suspension dans les peintures.

15. Application du polysaccharide selon la revendication 1, en tant qu'agent épaississant, agent gélifiant, agent de suspension, agent texturant et agent rétenteur d'eau.

## Claims

1. A polysaccharide having a repeat unit which has a side chain and comprises six neutral sugars including glucose and galactose and an acid sugar, a water solution or a saline solution containing 0,2 % or more by weight of the polysaccharide forming an elastic, transparent gel.

2. A polysaccharide according to claim 1, **characterised in that** sitting into solution said polysaccharide is promoted by heating said solution at a temperature in excess of 60°C.

3. A micro-organism capable of synthesising a polysacccharide according to any of claims 1 to 2, **characterised in that** it is Rhizobium deposited under number 1-1809 at the CNCM.

4. A method of producing a polysaccharide according to claim 1, **characterised in that** it comprises the following steps:
a) cultivation of a micro-organism which is a Rhizobium deposited under number 1-1809 at the CNCM on a medium rich in glucose and in nitrogenous substance,
b) recovery of the fermentation must,
c) directly obtaining the crude polysaccharide by precipitation

5. A method according to claim 4, **characterised in that** the fermentation must from step b) is processed as follows :
d) heat-treatment of the must after dilution at a temperature between 70 and 95°C,
e) centrifuging,
f) filtering the supernatant from step d), for example by frontal filtration,
g) processing the filtrate to obtain the polysacharide in dry form.

6. A method according to claim 4, **characterised in that** in step a) two culture media are used, called the precultivation medium and the production medium.

7. A method according to claim 6, **characterised in that** the « preculture » medium has the following composition :
| | |
|---|---|
| Glucose (sterilised separately) | 20 g |
| Yeast extract | 2,5 g |
| Ammonium | 1 g |
| Mineral solution (1) | 70 ml |
| Buffer solution (2) | 20 ml |
| Osmosed water | qs ad 1 litre |
| Inoculum | 7,5 % |

8. A method according to claim 6, **characterised in that** the composition of the production medium is as follows:
| | |
|---|---|
| Glucose | 20 g |
| Yeast extract | 1,7 g |
| Mineral solution (1) | 70 ml |
| Osmosed water | qs ad 1 litre |

9. Application of the polysaccharide according to any of claims 1 to 2 as a moisturising agent (alone or mixed with other already-known moisturising agents such as hyaluronic acid), a thickener, a gelling agent, or a suspension agent in cosmetics.

10. Application of the polysaccharide according to any of claims 1 to 2 as a gelling agent or suspension agent in detergents.

11. Use of the polysaccharide according to any of claims 1 to 2 as a texturising agent or suspension agent in food products.

12. Use of the polysaccharide according to any of claims 1 to 2 as a gelling agent in gelose fermentation media.

13. Use of the polysaccharide according to any of claims 1 to 2 as a soil aggregation agent, a water-retainging agent and a seed-coating agent in agriculture.

14. Use of the polysaccharide according to any of claims 1 to 2 as a texturing agent, a thickner or an agent for suspensions in paint.

15. Use of the polysaccharide according to claim 1 as a thickener, a gelling agent, a suspension agent, a texturing agent and a water-retaining agent.

## Patentansprüche

1. Polysaccharid mit einer Wiederholeinheit, welche eine Seitenkette aufweist und aus sechs neutralen Zuckern, darunter Glucose und Galactose, und einem sauren Zucker zusammengesetzt ist, wobei Pyruvat- und Acetat-Substituenten vorhanden sind, und wobei eine Lösung in Wasser oder eine Salzlösung mit einer Konzentration dieses Polysaccharids von mehr als 2 g/l ein elastisches und transparentes Gel bildet.

2. Polysaccharid nach Anspruch 1, **dadurch gekennzeichnet,**
**dass** das Lösen des Polysaccharids durch eine Erwärmung der Lösung auf eine Temperatur von mehr als 60° C begünstigt wird.

3. Mikroorganismus, der in der Lage ist, ein Polysaccharid nach einem der Ansprüche 1 bis 2 zu synthetisieren, **dadurch gekennzeichnet,**
**dass** es sich um ein unter der Nr. I-1809 bei der CNCM eingetragenes Rhizobium handelt.

4. Verfahren zur Herstellung eines Polysaccharids nach Anspruch 1,
**gekennzeichnet durch** folgende Schritte:
a) Kultur eines Mikroorganismus, welcher ein unter der Nr. I-1809 bei der CNCM eingetragenes Rhizobium ist, auf einem an Glucose und an Stickstoff reichen Medium,
b) Gewinnung des Fermentationsprodukts,
c) direkter Erhalt des Roh-Polysaccharids **durch** Fällung.

5. Verfahren nach Anspruch 4, **dadurch gekennzeichnet,**
**dass** das aus Schritt b) erhaltene Fermentationsprodukt folgendermaßen behandelt wird:
d) Wärmebehandlung des genannten Produkts, nach Verdünnung, bei einer Temperatur zwischen 70° C und 95° C,
e) Zentrifugieren,
f) Filtern der aus Schritt d) erhaltenen Aufschwemmung, beispielsweise durch frontales Filtern,
g) Behandlung des Filtrats, um das Polysaccharid in trockener Form zu erhalten.

6. Verfahren nach Anspruch 4, **dadurch gekennzeichnet,**
**dass** in Schritt a) zwei Kulturmedien verwendet werden: ein so genanntes Vorkulturmedium und ein so genanntes Produktionsmedium.

7. Verfahren nach Anspruch 6, **dadurch gekennzeichnet,**
**dass** das so genannte Vorkulturmedium folgende Zusammensetzung aufweist:
| | |
|---|---|
| Glucose (getrennt sterilisiert) | 20 g |
| Hefeextrakt | 2,5 g |
| Ammoniumsulfat | 1 g |
| Minerallösung (1) | 70 ml |
| Pufferlösung (2) | 20 ml |
| osmotisches Wasser | in für 1 l ausreichender Menge |
| Inoculum | 7,5%. |

8. Verfahren nach Anspruch 6, **dadurch gekennzeichnet,**
**dass** das so genannte Produktionsmedium folgende Zusammensetzung aufweist:
| | |
|---|---|
| Glucose | 20 g |
| Hefeextrakt | 1,7 g |
| Minerallösung (1) | 70 ml |
| osmotisches Wasser | in für 1 l ausreichender Menge. |

9. Anwendung des Polysaccharids nach einem der Ansprüche 1 bis 2 als Hydrationsmittel (allein oder in Mischung mit anderen, bereits bekannten Hydrationsmitteln wie etwa Hyaluronsäure), Verdickungsmittel, Geliermittel, Suspensionsmittel auf dem Gebiet der Kosmetik.

10. Anwendung des Polysaccharids nach einem der Ansprüche 1 bis 2 als Geliermittel, Suspensionsmittel auf dem Gebiet der Waschmittel.

11. Anwendung des Polysaccharids nach einem der Ansprüche 1 bis 2 als Strukturfestigungsmittel und Suspensionsmittel in Nahrungsmitteln.

12. Anwendung des Polysaccharids nach einem der Ansprüche 1 bis 2 als Geliermittel in Fermentationsmedien mit Nährböden.

13. Anwendung des Polysaccharids nach einem der Ansprüche 1 bis 2 als Bodenaggregationsmittel, Wasserhaltemittel und Umhüllungsmittel für Saatgut auf dem Gebiet der Landwirtschaft.

14. Anwendung des Polysaccharids nach einem der Ansprüche 1 bis 2 als Strukturfestigungsmittel, Verdickungsmittel und Suspensionsmittel in Anstrichstoffen.

15. Anwendung des Polysaccharids nach Anspruch 1 als Verdickungsmittel, Geliermittel, Suspensionsmittel, Strukturfestigungsmittel und Wasserhaltemittel.
